# FASCICULE DE BREVET EUROPEEN

(11) **EP 3 262 095 B1**
(45) Date de publication et mention de la délivrance du brevet: **26.12.2018**
(21) Numéro de dépôt: 16712944.4
(22) Date de dépôt: 17.02.2016
(51) Int. Cl.: C08G 63/06, C07C 51/363, C07C 51/367, C08G 63/78, C12P 7/52, C12P 7/54, C12P 7/64, C12P 7/40, C12P 7/42, C12P 7/62

(54) **PROCEDE DE PRODUCTION DE POLYHYDROXYALCANOATES A PARTIR DE PRECURSEURS OBTENUS PAR FERMENTATION ANAEROBIE A PARTIR DE BIOMASSE FERMENTESCIBLE**
VERFAHREN ZUR HERSTELLUNG VON POLYHYDROXYALKANOATEN AUS VORLÄUFERN DURCH ANAEROBE GÄRUNG AUS VERGÄRBARER BIOMASSE
PROCESS FOR PRODUCING POLYHYDROXYALKANOATES FROM PRECURSORS OBTAINED BY ANAEROBIC FERMENTATION FROM FERMENTABLE BIOMASS

(30) Priorité: 27.02.2015 FR 1551672
(43) Date de publication de la demande: 03.01.2018
(73) Titulaire: Afyren, 63360 Saint-Beauzire (FR)
(72) Inventeur: NOUAILLE, Régis, 63800 Cournon D'auvergne (FR); PESSIOT, Jérémy, 58400 La Charite Sur Loire (FR); THIEULIN, Marie, 10120 Saint Andre Les Vergers (FR)
(74) Mandataire: Dennemeyer & Associates S.A.
(86) Numéro de dépôt international: PCT/FR2016/050363
(87) Numéro de publication internationale: WO 2016/135396

(56) Documents cités:
- EP-A1- 0 110 761
- WO-A1-03/062439
- WO-A1-2014/170603
- WO-A2-2013/022998
- DE-A1- 2 810 975
- US-A- 5 686 275
- COLON G ET AL: "On-line removal of volatile fatty acids from CELSS anaerobic bioreactor via nanofiltration", LIFE SUPPORT & BIOSPHERE SCIENCE : INTERNATIONAL JOURNAL OF EARTH SPACE, COGNIZANT COMMUNICATION CORP. ELMSFORD, NY, US, vol. 7, no. 4, 1 janvier 2001 (2001-01-01) , pages 291-299, XP008174690, ISSN: 1069-9422

## Description

La présente invention concerne un procédé de production de polyhydroxyalcanoates à partir de précurseurs obtenus par fermentation anaérobie à partir de biomasse fermentescible.

Par la suite, pour faciliter la lecture, les polyhydroxyalcanoates seront désignés par leur acronyme : PHA. Cette famille comprend plusieurs molécules, en fonction du nombre de carbones.

Les PHA sont des polyesters thermoplastiques susceptibles d'être produits par fermentation microbienne à partir de sucres d'origine végétale, en particulier d'amidon de maïs ou de déchet végétal. Les microorganismes produisent des PHA lorsque se rencontrent des conditions de carence en certains métabolites associées à une offre excédentaire en carbone. En d'autres termes, les microorganismes accumulent alors le carbone, provenant des sucres, sous forme de granulés de PHA. Les PHA sont utilisés, par exemple, comme matériau constitutif des emballages ou dans le domaine médical comme matériel de suture. Ils se substituent aux polymères dérivés du pétrole.

On connait des procédés de fermentation utilisant des souches pures sélectionnées de microorganismes ainsi qu'un substrat spécifique pour produire des PHA. Une telle solution est difficilement exploitable à une échelle industrielle pour un coût de production acceptable.

On connait également par EP-A-2 749 650 un procédé adapté à une utilisation industrielle. Le substrat utilisé est issu d'effluents et enrichi en acides gras volatils (AGV), connus pour être des précurseurs des PHA. En utilisant un consortium bactérien et en alternant des phases de croissance de la biomasse et des phases de carence, de manière contrôlée, on stimule la croissance bactérienne et la production de PHA. Cependant un tel procédé implique un contrôle précis des conditions de fermentation et un traitement adapté pour extraire les PHA produits, ce qui implique une installation relativement lourde. Par ailleurs, une telle extraction ne peut se faire en continu et elle ne permet pas une production de tout type de PHA. Un autre inconvénient de ce procédé est sa spécificité : il ne peut produire que des PHA microbiens, c'est-à-dire produits par des microorganismes. Le document WO 03/062439 divulgue un procédé destiné à convertir des déchets organiques en matériaux thermoplastiques biodégradables contenant des polyhydroxyalkanoates (PHA). Ce procédé consiste à traiter les déchets organiques au moyen d'une population microbienne acidogène en vue de former des acides organiques volatils, et à polymériser ces acides organiques au moyen des espèces microbiennes produisant du PHA en vue de former des PHA.

L'invention vise plus particulièrement à remédier à ces inconvénients en proposant un procédé de production de PHA permettant de produire divers types de PHA, de manière aisée et sans les contraintes liées aux modes de production connus de l'état de l'art.

A cet effet, l'invention a pour objet un procédé de production de polyhydroxyalcanoates ou PHA à partir de molécules d'acides gras volatils (AGV), dites précurseurs, produites par fermentation anaérobie à partir de biomasse fermentescible, caractérisé en ce qu'il comprend au moins les étapes suivantes :
- a) extraire les molécules d'acides gras volatils (AGV), sans interruption de la fermentation, par un moyen d'extraction choisi parmi des moyens qui sont, au moins, insolubles dans le milieu de fermentation,
- b) collecter, en dehors du réacteur de fermentation, les molécules d'acides gras volatils (AGV) une fois extraites,
- c) synthétiser, par halogénation, à partir d'un type d'acide gras volatil (AGV) choisi parmi les acides gras volatils collectés à l'étape b) et défini selon le type de PHA voulu, un acide α-halogéné donné,
- d) synthétiser à partir de cet acide α-halogéné des molécules d'un α-hydroxyacide donné par réaction avec une base,
- e) polymériser à partir de l'α-hydroxyacide obtenu un polyhydroxyalcanoate (PHA) défini.

Ainsi, un tel procédé permet de coupler une phase de production en continu de précurseurs par des microorganismes avec une phase de synthèse réalisée hors fermentation, ce qui permet un contrôle aisé des différents paramètres, tout en autorisant une plus grande variabilité dans le type de polyhydroxyalcanoates (PHA) produits.

Un tel procédé permet de disposer, en continu, de précurseurs, à savoir des acides gras volatils, tout en préservant la capacité de production des microorganismes présents dans le bioréacteur.

En effet, les étapes a) et b) d'extraction et de collecte permettent non seulement d'extraire et de collecter en continu les molécules d'acides gras volatils produites dans le réacteur de fermentation mais également de préserver les microorganismes responsables de cette production. En effet, l'extraction, et de facto la collecte, est effectuée dans des conditions au moins non létales pour la totalité des microorganismes, c'est-à-dire dans des conditions d'extraction et de collecte biocompatibles, cela du fait que l'extraction préserve l'activité des microorganismes et que la collecte s'effectue en dehors du réacteur de fermentation.

De cette manière, on s'affranchit des problèmes liés à l'accumulation des métabolites dans le réacteur de fermentation, par exemple de l'acidification du milieu de fermentation par accumulation des acides gras volatils produits qui sont nocifs pour les microorganismes. On maintient à un niveau élevé, proche du niveau initial, la quantité et l'activité des microorganismes tout au long du cycle de fermentation.

En disposant d'une production continue et régulière d'AGV, on a une source de précurseurs variés aisément utilisable et de manière rapide. Dans le procédé objet de l'invention, cette utilisation se fait, à partir de l'étape c), par synthèse chimique et donc dans des conditions aisément contrôlables et modifiables, cela en offrant de plus une grande variabilité dans le type de molécules synthétisées. En effet, lors de l'étape c), selon l'AGV retenu pour effectuer l'halogénation, on obtient un type donné d'acide α-halogénés et donc, par la suite, un type défini d'a-hydroxyacides et donc un type donné de α-PHA. L'invention permet également de combiner plusieurs α-hydroxyacides ainsi obtenus pour produire, lors de la dernière étape du procédé, des hétéropolymères.

Il existe plusieurs types de PHA présentant un intérêt pour une utilisation industrielle, cosmétique, médicale, alimentaire ou autre. A titre d'exemple on peut citer le polyhydroxybutyrate ou PHB, le poly(3-hydroxybutyrate-co3-hydroxyvalérate)ou PHBV. On peut également citer, parmi les types de PHA, la production de polyacide glycolique ou PGA ou de polyacide lactique ou PLA, sachant que, par le procédé connu de l'état de la technique, il n'est pas possible de produire directement le PLA, ce dernier doit être polymérisé à partir d'une production par fermentation d'acide lactique.

En d'autres termes, grâce à l'invention, on peut réaliser la synthèse de plusieurs types de PHA, à savoir de types homo, co ou hétéropolymères, de manière régulière et contrôlée, à partir d'un substrat bio-sourcé en associant une production par voie biologique avec une production par voie chimique.

Un tel procédé permet, lors de la phase de fermentation anaérobie d'utiliser de la biomasse fermentescible. Par biomasse fermentescible, on désigne ici un substrat organique, avantageusement non alimentaire, obtenu à partir de déchets, sous-produits et coproduits formés de matières organiques, c'est-à-dire de la biomasse, issue des activités humaines, qu'elles soient domestiques, industrielles, agricoles, forestières, aquacoles, agro-industrielles, issue de l'élevage ou autre. A titre d'exemple non limitatif, on peut citer comme substrat organique les fumiers, la fraction organique des ordures ménagères, les coproduits d'abattoir, des résidus cellulosiques ou ligno-cellulosiques provenant de l'agro-industrie tels ceux issus de la transformation de la canne à sucre (bagasse), du tournesol ou du soja.

Par fermentation anaérobie on entend une fermentation réalisée dans des conditions anaérobies par des microorganismes, eucaryotes ou procaryotes, tels que des bactéries, des champignons, des algues ou des levures.

Selon des aspects avantageux mais non obligatoires de l'invention, un tel procédé peut comprendre une ou plusieurs des caractéristiques suivantes:
- Lors de l'étape c), le composé halogéné utilisé est du dibrome.
- Lors de l'étape c), le composé halogéné utilisé est différent du dibrome.
- Lors de l'étape c), on utilise de l'anhydride acétique dans un pourcentage molaire par rapport à l'acide gras volatil voisin de 12%.
- Lors de l'étape c), on utilise un anhydride correspondant à l'acide gras volatil (AGV) à halogèner.
- Lors de l'étape c), la température à laquelle la réaction de bromation est réalisée est inférieure de 20°C à 40°C à la température d'ébultion de l'acide gras volatil.
- Lors de l'étape d), la base utilisée est la soude.
- Lors de l'étape d), la soude est en quantité équimolaire avec l'acide α-halogéné.
- Lors de l'étape d), la réaction de l'acide α-halogéné avec la soude est réalisée entre 20°C et 120°C, avantageusement entre 50°C et 90°C.
- Lors de l'étape d), la réaction de l'acide α-halogéné avec la soude est optimale pour une température voisine de 50°C si l'acidea-halogéné a au moins quatre carbones et pour une température voisine de 90°C si l'acidea-halogéné a moins de quatre carbones.

L'invention sera mieux comprise et d'autres avantages de celle-ci apparaitront plus clairement à la lecture de la description de plusieurs modes de réalisation de l'invention, donnée à titre d'exemple non limitatif.

Les différentes étapes du procédé sont maintenant décrites en référence à plusieurs modes de réalisation, étant entendu que les étapes connues en soi ne sont pas détaillées.

Tout d'abord le substrat utilisé est, avantageusement, non traité, à savoir qu'il n'a subi aucun prétraitement physico-chimique ou enzymatique. Ce substrat est majoritairement constitué par de la biomasse fermentescible. A titre d'exemple complémentaire non limitatif, on peut citer les déchets agricoles ou végétaux (paille, bagasse, drèche de maïs, herbes, bois, tontes) les déchets papetiers (carton, papier), les déchets agroalimentaires, les déchets d'abattoirs, la fraction organique des ordures ménagères, les effluents d'élevage (fumiers, lisiers, fientes), les algues, les déchets d'aquaculture, les déchets d'activité forestière ou encore les coproduits fermentescibles de l'industrie cosmétique. Certains substrats contiennent des molécules organiques, telles des acides organiques, qui n'influeront pas, ou de façon marginale, sur le procédé de fermentation. En revanche, ces molécules peuvent se retrouver dans le milieu de fermentation et participer, par exemple, à la production des molécules organiques finales définies.

Pour mémoire et de façon connue, le substrat est introduit dans un réacteur de fermentation, connu en soi et dimensionné pour la production souhaitée, que cette dernière soit à l'échelle du laboratoire pour effectuer des essais ou à l'échelle industrielle dans le cas d'une production. En d'autres termes, le réacteur de fermentation ou bioréacteur a un volume variant de quelques litres à plusieurs centaines de mètres cubes, selon les besoins.

Des microorganismes sont, avantageusement, introduits initialement dans le réacteur de fermentation, en quantité suffisante pour débuter la fermentation. Les microorganismes sont, avantageusement, inoculés sous forme d'un consortium. Par le terme consortium, on désigne un mélange ou mix de microorganismes, eucaryotes et procaryotes, qu'il s'agisse de bactéries, levures, champignons ou algues. Ces différents microorganismes proviennent essentiellement d'écosystèmes naturels, avantageusement mais non exclusivement, d'écosystèmes anaérobies tels que, à titre d'exemple non limitatif, la zone anaérobie des milieux aquatiques telle la zone anoxique de certains lacs, les sols, les marais, les boues d'épuration, le rumen des ruminants ou l'intestin des termites. Il convient de garder à l'esprit que la distribution qualitative et quantitative des différents types et espèces de microorganismes dans le consortium n'est pas connue précisément et surtout peut varier dans des proportions importantes. Il s'avère que cette diversité qualitative et quantitative apporte de façon surprenante une robustesse et une adaptabilité des microorganismes qui permettent d'assurer une utilisation optimale des substrats, quel que soit la composition de ces derniers et cela dans des conditions de fermentation variables.

Par ailleurs, du fait que le substrat est utilisé tel quel, c'est-à-dire qu'il n'est pas stérilisé ou, plus généralement, qu'il n'est pas débarrassé des microorganismes qu'il contient préalablement à son introduction dans le bioréacteur, il s'avère que les microorganismes endémiques au substrat sont, de facto, incorporés dans le consortium ou du moins associés à ce dernier dans le bioréacteur.

Par ailleurs, la fermentation a lieu en conditions anaérobies, plus précisément lorsque le potentiel redox est inférieur à -300mV, avantageusement compris entre -550mV et -400mV et lorsque le pH est inférieur à 8, de préférence compris entre 4 et 7. La fermentation est, avantageusement, limitée à la production de métabolites fermentaires dits précurseurs, à savoir des acides gras volatils ou AGV ayant de deux à huit carbones, préférentiellement de deux à six. On induit ainsi une réaction similaire au phénomène d'acidose rencontré chez les ruminants tout en ayant une production de méthane proche de zéro. Le méthane est, généralement, un des métabolites fermentaires finaux obtenus lors d'une fermentation anaérobie par des microorganismes issus d'écosystèmes naturels.

La fermentation conduit, dans un premier temps, à la formation d'acides gras volatils ayant, principalement, de deux à quatre carbones tels que, par exemple, l'acide acétique, l'acide propionique et l'acide butyrique. On obtient aussi, en quantité moindre, des acides gras volatils à longue chaine, donc supérieure à quatre carbones, tels que les acides valérique et caproïque, heptanoïque ou octanoïque. En poursuivant la fermentation et/ou en augmentant la quantité de microorganismes dans le bioréacteur, si besoin avec des microorganismes sélectionnés, il est possible de favoriser la production d'AGV à longue chaine carbonée, donc supérieure à quatre carbones.

En d'autres termes, les acides gras volatils produits en quantité lors de la fermentation sont essentiellement des acides gras volatils de deux à six carbones.

La fermentation est, dans tous les cas, menée pour assurer la production d'AGV, en phase liquide. Typiquement, la période de fermentation est comprise entre 1 et 7 jours, préférentiellement entre 2 et 4 jours. La concentration en métabolites obtenue dans le milieu de fermentation à l'issue de cette période est variable, mais, pour des acides gras volatils, est généralement de l'ordre de 10 à 20 g/L, selon les acides gras volatils, étant entendu que dans certaines conditions elle peut être supérieure à 35 g/L, par exemple voisine de 50 g/L. A la fin de l'étape de fermentation, le milieu de fermentation est à un pH acide, qui est généralement compris entre 4 et 6, du fait de la présence des acides gras volatils dans le milieu de fermentation.

Lorsque la production en AGV atteint une quantité définie, généralement en phase de régime permanent de la fermentation, l'étape a) d'extraction des molécules est initiée. De manière préférée mais non obligatoire, cette quantité définie d'AGV correspond à un ralentissement de la croissance des microorganismes, donc au voisinage d'un seuil d'inhibition des microorganismes.

Le moyen d'extraction est choisi parmi des moyens d'extraction, liquide ou solide, qui sont, au moins, insolubles dans le milieu de fermentation. Lorsque le moyen d'extraction est liquide, donc lorsqu'il s'agit d'un solvant, préférentiellement, la densité du solvant est inférieure à celle du milieu de fermentation.

De manière plus précise, l'extraction est conduite avec un moyen d'extraction, solide ou liquide, dont les conditions de mises en oeuvre permettent de préserver l'activité et/ou la croissance des microorganismes dans les conditions de fermentation régnant dans le bioréacteur et qui sont définies pour réaliser la fermentation. Les molécules d'AGV sont préférentiellement extraites par familles moléculaires puis avantageusement séparées individuellement par des techniques connues en soi.

Lorsque l'on extrait du milieu de fermentation des molécules telles que des acides gras volatils, de facto on réduit l'acidification du milieu de fermentation par ces acides. Ainsi, la fermentation, et donc la production de métabolites, se poursuit dans des conditions similaires aux conditions initiales, le milieu de fermentation restant peu acide.

L'extraction est, avantageusement, conduite en continu ou au moins de manière séquentielle, par exemple avec une extraction toutes les 12 heures. En d'autres termes, il est possible de poursuivre la fermentation tout en extrayant les métabolites produits, soit au fur et à mesure de leur production soit de manière régulière.

L'extraction liquide-liquide avec des solvants organiques comme moyen d'extraction est le mode d'extraction, préférentiellement mais non exclusivement, retenu.

Dans un mode de réalisation, l'extraction n'est pas réalisée dans un organe distinct du réacteur de fermentation mais directement dans ce dernier. Le solvant étant, par exemple, introduit par un dispositif de type bulleur situé en partie basse du réacteur. En variante, un organe d'extraction est couplé avec le réacteur, une communication avec le milieu de fermentation étant ménagée.

A l'issue de l'extraction, l'étape b) de collecte est mise en oeuvre. Lors de cette étape, les AGV sont collectés à partir de la phase organique par des techniques connues en soi, telles que la distillation ou l'évaporation.

La collecte est effectuée soit en mélange d'AGV soit par type d'AGV. On conçoit que le choix de l'AGV ou du mélange d'AGV est déterminé par le type de molécule(s) finale(s) souhaitée(s). Pour cela, les conditions de collecte, typiquement les paramètres d'évaporation ou de distillation, sont adaptées.

Une fois cette étape de collecte réalisée, on effectue l'étape c) suivante. Celle-ci est, avantageusement mais non exclusivement, effectuée à la suite de l'étape de collecte. En variante, elle est réalisée à un autre moment et/ou un autre endroit, les AGV produits étant transportés et/ou stockés, selon des techniques connues en soi.

Cette étape d'halogénation consiste à faire agir un halogène avec un AGV afin de produire un acide α-halogéné qui est un type de molécule très réactive et donc particulièrement intéressante pour produire d'autres molécules. Une telle réaction, connue en soi, est effectuée par ajout de brome, cela de manière préférée, étant entendu que l'on peut utiliser les autres halogènes, à savoir le chlore, le fluor ou l'iode ou des molécules halogénées telles que les trihalogénures de phosphore, les acides halogénés ou les halogénures d'acyle.

Le dibrome a été retenu car un α-haloacide bromé est plus réactif que l'a-haloacide chloré correspondant, une liaison carbone-brome étant plus facile à rompre qu'une liaison carbone-chlore. De plus, le dibrome est plus aisé à manipuler du fait de sa forme liquide.

Pour réaliser la synthèse d'acide α-bromé la voie utilisant un anhydride, ici de l'anhydride acétique, et de la pyridine a été retenue. On conçoit que d'autres voies de synthèse, par exemple avec de l'acide polyphosphorique ou les trihalogénures de phosphore, sont connues en soi. Des essais avec de l'acide polyphosphorique ont été menés mais les résultats n'ont pas été concluants, entre autre du fait de la viscosité élevée de ce composé qui rend sa manipulation difficile.

Des essais de chloration ont également été menés par la demanderesse pour la synthèse d'acides α-chlorés, par exemple avec de l'acide trichloroisocyanurique. Les résultats obtenus sont inférieurs, en termes de rendement et de facilité de mise en oeuvre à ceux obtenus avec le dibrome.

La voie de synthèse mettant en oeuvre un anhydride correspondant à l'acide gras volatil que l'on veut halogéner est intéressante et permet d'obtenir un acide α-halogéné, ici un acide α-bromé d'un type donné. L'utilisation de l'anhydride acétique avec d'autres AGV et/ou sur un mélange d'AGV de deux à six carbones permet d'obtenir un mélange d'acides α-halogénés de deux à six carbones.

Des essais mettant en oeuvre de l'acide acétique (AGV à deux carbones), de l'acide propionique (AGV à trois carbones), de l'acide butyrique (AGV à quatre carbones), de l'acide caproïque (AGV à six carbones) ainsi qu'un mélange d'AGV de deux à six carbones ont été réalisés en faisant varier la quantité d'anhydride acétique ainsi que d'autres paramètres tels que la température.

Lors des différents essais, un protocole est respecté. Il s'agit, en phase préliminaire, de chauffer à reflux un mélange initial d'AGV, d'anhydride acétique et de pyridine. Ensuite, lors de la bromation proprement dite, le dibrome est ajouté lentement, pendant plusieurs heures, à une température inférieure à la température d'ébullition du mélange, une fois le dibrome ajouté, le mélange est porté à nouveau à reflux avant d'être refroidi. En fin de réaction, avantageusement, de l'eau est ajoutée pour détruire l'anhydride présent. L'acide α-bromé est extrait ensuite par différentes méthodes, selon l'acide. Il s'agit par exemple, de distillation, d'extraction séparative.

La température initiale, pour porter le mélange à reflux, est comprise entre 120°C, pour les AGV à deux carbones et 200°C, pourles AGV à six carbones. La température de bromation varie de 80°C à 180°C, selon que les AGV ont de deux à six carbones. Le temps de la réaction de bromation, donc de facto le temps d'ajout du dibrome, varie d'environ une heure pour les AGV à six carbones à environ quatre heures pour les AGV à deux carbones.

Des essais de bromation des acides gras volatils à deux, trois, quatre, six carbones ont été effectués ainsi qu'un essai sur un mélange d'acides gras volatils:
Acide acétique (C2) : 0,53 mol
Acide propionique (C3) : 0,53 mol
Acide butyrique (C4) : 0,53 mol
Acide caproïque (C6) : 0,24 mol
Mélange d'AGV de C2 à C6 : 0,54 mol.

La quantité de dibrome ajoutée est 0,21 mol ou 0,11 mol de telle sorte que l'acide gras volatil soit en excès. Avantageusement, la demanderesse a constaté qu'un rapport molaire de 2 :1 en faveur de l'AGV est optimal.

La quantité d'anhydride ajoutée est, pour chaque acide, de 0,06 mol pour un essai et de 0,03 mol pour un autre essai. Le mélange d'AGV comprend les acides acétique (C2), propionique (C3), butyrique (C4), valérique (C5) et caproïque (C6).

Les températures de reflux, lors de la phase préliminaire varient selon l'AGV : 120°C pour l'acide acétique ; 120°C et 140°C pour les essais avec l'acide propionique ; 150°C et 160°C pour l'acide butyrique ; 200°C pour l'acide caproïque et 180°C pour le mélange.

Les températures de bromation pour les différents essais avec chaque acide sont inférieures de 10°C à 50°C, et avantageusement de 20°C à 40°C, aux températures de reflux, donc d'ébullition de l'acide gras volatil.

Les rendements et les puretés des acides α-bromés obtenus à l'issue des différents essais sont repris ci-dessous dans le tableau 1. Les AGV sont, pour simplifier, désignés par le nombre de carbones.

**TABLEAU 1**

| Acides | Quantité anhydride (mol) | Temps de bromation (h) | Température de reflux (°C) | Température de bromation (°C) | Rendement (%) | Pureté (%) |
|---|---|---|---|---|---|---|
| C2 | 0,06 | 2,2 | 120 | 100 | 87 | 98 |
| C2 | 0,03 | 4 | 120 | 90 | 80 | 93 |
| C3 | 0,06 | 3 | 120 | 80 à 110 | 77 | 80 |
| C3 | 0,03 | 3 | 140 | 125 | 100 | 93 |
| C4 | 0,06 | 1,25 | 160 | 140 | 80 | 95 |
| C4 | 0,03 | 3 | 150 | 110 à 140 | 100 | 96 |
| C6 | 0,03 | 0,92 | 200 | 150 | 100 | NC |
| mélange | 0,06 | 1,5 | 180 | 130 | 100 | NC |

L'analyse et les calculs de rendement ont été réalisés par des techniques analytiques connues en soi, à savoir par RMN (Résonnance Magnétique Nucléaire) et par HPLC (High Performance Liquid Chromatography). Les rendements sont définis par rapport à la quantité d'AGV consommée.

La demanderesse a constaté que la vitesse de la réaction, illustrée par la décoloration du mélange réactionnel après ajout du dibrome, est plus rapide lorsque la quantité d'anhydride est plus importante, la pureté étant peu affectée. Néanmoins, il convient que la température des deux étapes, préliminaire et de bromation, soit optimale. Pour cela, la demanderesse a noté qu'une température de bromation inférieure à la température d'ébullition de l'acide gras volatil est nécessaire, cela sans être trop éloignée de cette température.

Les différents essais ont permis de définir qu'une température de bromation inférieure d'environ 10°C à 50°C à la température d'ébullition de l'acide gras volatil et, avantageusement, inférieure de 20 °C permettait, toute autre condition étant identique, d'obtenir un rendement optimal, typiquement entre 60% et 100% avec un temps de réaction de 1h à 4h.

Concernant le rôle de l'anhydride acétique, à la vue des résultats du tableau, il apparait que le pourcentage molaire d'anhydride par rapport à l'AGV doit être voisin de 12% pour une réaction de bromation optimale, étant entendu qu'un pourcentage compris entre 5% et 20% est acceptable.

A partir des acides α-bromés obtenus, ou plus précisément à partir d'un acide α-bromé donné, on réalise ensuite la synthèse, lors de l'étape d), d'un acide α-hydroxylé, également dénommé α-hydroxyacide, donné. Pour cela on ajoute une base. Avantageusement, mais non exclusivement, il s'agit de la soude.

Des essais ont été effectués par la demanderesse par substitution de la soude (NaOH) sur des α-bromoacides, à savoir sur l'acide bromoacétique, l'acide α-bromopropionique, l'acide α-bromobutyrique, sur l'acide α-bromocaproïque et sur un mélange de α-bromoacides ayant de deux à six carbones. Une telle substitution permet l'obtention d'acides α-hydroxylés ayant une chaine carbonée respectivement de deux, trois, quatre, cinq ou six carbones, soit l'acide glycolique, l'acide lactique, l'acide α-hydroxybutyrique, l'acide α-hydroxyvalérique ou l'acide α-hydroxycaproïque.

Ces acides α-hydroxylés sont parmi les plus utilisés pour, après polymérisation, produire des cosmétiques ou des emballages alimentaires. On conçoit aisément que le procédé objet de l'invention permet la production d'autres types d'acides qui permettent la polymérisation d'autres types de PHA. A titre d'exemple, on peut citer l'acide α-hydroxydécanoïque.

Pour les différents essais, le protocole a consisté à porter à reflux un mélange équimolaire de soude et d'acide α-bromé pendant une durée d'une à deux heures. La température de chauffage varie de 80°C à 120°C. Des prélèvements à intervalles réguliers ont permis de montrer que le rendement est compris entre 60% et 100% et, avantageusement entre 80% et 100% pour les acides ayant au moins trois carbones.

Il apparait également que le rendement optimal est atteint pendant la montée progressive de la température pour les composés ayant plus de quatre carbones. La demanderesse a ainsi constaté que le rendement est optimal lorsque la température reste inférieure à une température à partir de laquelle il y a un début de dégradation de l'acide α-hydroxylé, cette température étant de facto inférieure à la température d'ébullition de l'acide α-bromé.

En particulier, la demanderesse a constaté, de manière surprenante, que la température à laquelle le rendement optimal est atteint est comprise entre 20°C et 120°C, avantageusement entre 50°C et 90°C.

La demanderesse a notamment constaté que, de façon inattendue, la température à laquelle le rendement optimal est atteint est voisine de 50°C pour les acides α-bromés ayant au moins quatre carbones et voisine de 90°C pour les acides α-bromés ayant moins de quatre carbones, cela étant entendu que, pour les acides C2 et C3 α-hydroxylés, une température supérieure à 100°C induit une dégradation du composé.

En effet, une fois le rendement optimal atteint, un allongement du temps de réaction ne procure qu'un gain minime, par exemple un gain de 4% de rendement pour une durée de réaction de deux heures à la place d'une heure pour un acide α-bromé à quatre carbones.

Le tableau 2 ci-dessous reprend certains des essais effectués.

**TABLEAU 2**

| Halo-acides | Mole acide | Mole NaOH | Température de chauffage (°C) | Temps de reflux (h) | Rendement (%) |
|---|---|---|---|---|---|
| BrC2 | 0,05 | 0,05 | 110-120 | 2 | 80 |
| BrC2 | 0,05 | 0,05 | 110-120 | 2 | 85 |
| BrC3 | 0,05 | 0,05 | 110-120 | 2 | 80 |
| BrC4 | 0,05 | 0,05 | 110-120 | 2 | 100 |
| BrC4 | 0,05 | 0,05 | 110-120 | 1 | 96 |
| BrC6+BrC2 | 0,041+0,0019 | 0,043 | 100 | 1 | 95 |
| BrC3+BrC2 | 0,05+0,014 | 0,064 | 100 | 2 | 62 |
| Mélange BrC2-BrC6 | 0,042 | 0,042 | 80 | 2 | 83 |

L'analyse et les calculs de rendements ont été réalisés par les techniques analytiques de RMN et d'HPLC, déjà citées. Les rendements sont définis par rapport à la quantité d'acide halogéné initiale.

La dernière étape du procédé consiste à polymériser le polyhydroxyalcanoate ou PHA correspondant à partir de l'a-hydroxyacide obtenu. A titre d'exemple, on peut citer le polyacide lactique ou PLA, qui est employé dans des emballages alimentaires, le polyacide glycolique ou PGA utilisé dans le domaine médical comme matériel de suture. Le PLA est issu de l'acide lactique obtenu à partir de l'acide α-bromopropionique. Le PGA est issu de l'acide glycolique obtenu à partir de l'acide bromoacétique.

Cette polymérisation, connue en soi, s'effectue avantageusement selon trois voies : par ouverture de cycle (Ring-Opening Polymerization), par polymérisation en phase solide (Solid State Polymerization) ou par une polycondensation directe (direct polycondensation). Cette polymérisation est suivie d'une récupération du PHA connue en soi.

## Revendications

1. Procédé de production de polyhydroxyalcanoates ou PHA à partir de molécules d'acides gras volatils (AGV), dites précurseurs, produites par fermentation anaérobie à partir de biomasse fermentescible, **caractérisé en ce qu'**il comprend au moins les étapes suivantes :
- a) extraire les molécules d'acides gras volatils (AGV), sans interruption de la fermentation, par un moyen d'extraction choisi parmi des moyens qui sont, au moins, insolubles dans le milieu de fermentation,
- b) collecter, en dehors du réacteur de fermentation, les molécules d'acides gras volatils (AGV) une fois extraites,
- c) synthétiser, par halogénation, à partir d'un type d'acide gras volatil (AGV) choisi parmi les acides gras volatils collectés à l'étape b) et défini selon le type de PHA voulu, un acide α-halogéné donné,
- d) synthétiser à partir de cet acide α-halogéné des molécules d'un α-hydroxyacide donné par réaction avec une base,
- e) polymériser à partir de l'α-hydroxyacide obtenu un polyhydroxyalcanoate (PHA) défini.

2. Procédé selon la revendication 1, **caractérisé en ce que**, lors de l'étape c), le composé halogéné utilisé est du dibrome.

3. Procédé selon la revendication 1, **caractérisé en ce que**, lors de l'étape c), le composé halogéné utilisé est différent du dibrome.

4. Procédé selon la revendication 1 ou 2, **caractérisé en ce que** lors de l'étape c), on utilise de l'anhydride acétique dans un pourcentage molaire par rapport à l'acide gras volatil voisin de 12%.

5. Procédé selon la revendication 1 ou 2, **caractérisé en ce que** lors de l'étape c), on utilise un anhydride correspondant à l'acide gras volatil (AGV) à halogéner.

6. Procédé selon une des revendications précédentes, **caractérisé en ce que** lors de l'étape c), la température à laquelle la réaction de bromation est réalisée est inférieure de 20°C à 40°C à la température d'ébullition de l'acide gras volatil.

7. Procédé selon une des revendications précédentes, **caractérisé en ce que**, lors de l'étape d), la base utilisée est la soude.

8. Procédé selon la revendication 7, **caractérisé en ce que**, lors de l'étape d), la soude est en quantité équimolaire avec l'acide α-halogéné.

9. Procédé selon la revendication 7 ou 8, **caractérisé en ce que** lors de l'étape d), la réaction de l'acide α-halogéné avec la soude est réalisée entre 20°C et 120°C, avantageusement entre 50°C et 90°C.

10. Procédé selon la revendication 9, **caractérisé en ce que** lors de l'étape d), la réaction de l'acide α-halogéné avec la soude est optimale pour une température voisine de 50°C si l'acide α-halogéné a au moins quatre carbones et pour une température voisine de 90°C si l'acidea-halogéné a moins de quatre carbones.

## Patentansprüche

1. Verfahren zur Herstellung von Polyhydroxyalkanoaten oder PHA aus als Vorläufer bezeichneten flüchtigen Fettsäuremolekülen, die durch anaerobe Gärung aus vergärbarer Biomasse hergestellt werden, **dadurch gekennzeichnet, dass** es mindestens die folgenden Schritte umfasst:
- a) Extrahieren der flüchtigen Fettsäuremoleküle, ohne die Gärung zu unterbrechen, durch ein Extraktionsmittel, das aus Mitteln ausgewählt ist, die mindestens in der Gärungsumgebung unlöslich sind,
- b) Auffangen der einmal extrahierten flüchtigen Fettsäuremoleküle außerhalb des Fermentationsreaktors,
- c) Synthetisieren einer bestimmten α-halogenierten Säure durch Halogenierung aus einer Art von flüchtiger Fettsäure, die aus den in Schritt b) aufgefangenen flüchtigen Fettsäuren ausgewählt ist und nach Art des gewünschten PHA definiert ist,
- d) Synthetisieren von Molekülen einer bestimmten α-Hydroxysäure aus dieser α-halogenierten Säure durch Reaktion mit einer Base,
- e) Polymerisieren eines definierten Polyhydroxyalkanoats (PHA) aus der erhaltenen α-Hydroxysäure.

2. Verfahren nach Anspruch 1, **dadurch gekennzeichnet, dass** bei Schritt c) die verwendete halogenierte Verbindung Dibrom ist.

3. Verfahren nach Anspruch 1, **dadurch gekennzeichnet, dass** sich bei Schritt c) die verwendete halogenierte Verbindung von Dibrom unterscheidet.

4. Verfahren nach Anspruch 1 oder 2, **dadurch gekennzeichnet, dass** bei Schritt c) Essigsäureanhydrid in einem molaren Prozentsatz im Verhältnis zur flüchtigen Fettsäure von etwa 12 % verwendet wird.

5. Verfahren nach Anspruch 1 oder 2, **dadurch gekennzeichnet, dass** bei Schritt c) ein der zu halogenierenden flüchtigen Fettsäure entsprechendes Anhydrid verwendet wird.

6. Verfahren nach einem der vorhergehenden Ansprüche, **dadurch gekennzeichnet, dass** bei Schritt c) die Temperatur, bei der die Bromierungsreaktion durchgeführt wird, 20°C bis 40°C unter der Siedetemperatur der flüchtigen Fettsäure liegt.

7. Verfahren nach einem der vorhergehenden Ansprüche, **dadurch gekennzeichnet, dass** bei Schritt d) die verwendete Base Soda ist.

8. Verfahren nach Anspruch 7, **dadurch gekennzeichnet, dass** bei Schritt d) das Soda in äquimolarer Menge mit der α-halogenierten Säure vorliegt.

9. Verfahren nach Anspruch 7 oder 8, **dadurch gekennzeichnet, dass** bei Schritt d) die Reaktion der α-halogenierten Säure mit dem Soda zwischen 20 °C und 120 °C, vorteilhafterweise zwischen 50 °C und 90 °C, durchgeführt wird.

10. Verfahren nach Anspruch 9, **dadurch gekennzeichnet, dass** bei Schritt d) die Reaktion der α-halogenierten Säure mit dem Soda optimal für eine Temperatur von etwa 50 °C ist, wenn die α-halogenierte Säure mindestens vier Kohlenstoffe enthält, und für eine Temperatur von etwa 90 °C, wenn die α-halogenierte Säure weniger als vier Kohlenstoffe enthält.

## Claims

1. A process for producing polyhydroxyalkanoates or PHAs from volatile fatty acid (VFA) molecules, termed precursors, produced by anaerobic fermentation from fermentable biomass, **characterised in that** said process comprises at least the following steps:
- a) extracting the volatile fatty acid (VFA) molecules without interrupting the fermentation using an extraction means chosen from among means which are, at least, insoluble in the fermentation medium,
- b) collecting, outside the fermentation reactor, the volatile fatty acid (VFA) molecules once extracted,
- c) synthesising a given α□haloacid by halogenation starting from a type of volatile fatty acid (VFA) chosen from among the volatile fatty acids collected in step b) and defined according to the type of PHA desired,
- d) synthesising, from this α□haloacid, molecules of a given α□hydroxyacid by reaction with a base,
- e) polymerising a defined polyhydroxyalkanoate (PHA) from the α□hydroxyacid obtained.

2. The process according to claim 1, **characterised in that** the halogen compound used during step c) is dibromine.

3. The process according to claim 1, **characterised in that** the halogen compound used during step c) is different from dibromine.

4. The process according to claim 1 or 2, **characterised in that**, during step c), acetic anhydride is used in a molar percentage close to 12% with respect to the volatile fatty acid.

5. The process according to claim 1 or 2, **characterised in that**, during step c), an anhydride corresponding to the volatile fatty acid (VFA) to be halogenated is used.

6. The process according to one of the preceding claims, **characterised in that**, during step c), the temperature at which the bromination reaction is carried out is less than 20 °C to 40 °C at the boiling temperature of the volatile fatty acid.

7. The process according to one of the preceding claims, **characterised in that**, during step d), the base used is soda.

8. The process according to claim 7, **characterised in that**, during step d), the soda is in equimolar quantity with the α-haloacid.

9. The process according to claim 7 or 8, **characterised in that**, during step d), the reaction of the α-haloacid with the soda is carried out between 20 °C and 120 °C, advantageously between 50 °C and 90 °C.

10. The process according to claim 9, **characterised in that**, during step d), the reaction of the α-haloacid with the soda is optimal for a temperature close to 50 °C if the α-haloacid has at least four carbon atoms, and for a temperature close to 90 °C if the α-haloacid has less than four carbon atoms.
